# EUROPEAN PATENT APPLICATION

(11) **EP 4 759 796 A2**
(43) Date of publication of application: **17.06.2026**
(21) Application number: 26167716.5
(22) Date of filing: 17.10.2022
(51) Int. Cl.: C07C 67/347

(54) **BIS-AMINOPHOSPHINES AS CATALYSTS FOR THE DIMERIZATION OF ALKYL ACRYLATES**

(30) Priority: 18.10.2021 EP 21203095
(62) Divisional of application: 22803223.1
(71) Applicant: SPECIALTY OPERATIONS FRANCE, 69003 Lyon (FR)
(72) Inventor: BACK, Olivier, 69190 Saint-Fons (FR)
(74) Representative: Valentino, Cédric

(57) **Abstract**

The present invention relates to bis-aminophosphines which are useful as catalysts for the dimerization of alkyl acrylates. Furthermore, the present invention relates to a process for preparing bis-aminophophines, the use of these bis-aminophosphines as catalysts for dimerization reaction of alkyl acrylates and a process for obtaining dimers of alkyl acrylates using bis-aminophosphines as catalyst.

## Description

### Field of the invention

The present invention relates to bis-aminophosphines which are useful as catalysts for the dimerization of alkyl acrylates. Furthermore, the present invention relates to a process for preparing bis-aminophophines, the use of these bis-aminophosphines as catalysts for dimerization reactions of alkyl acrylates and a process for obtaining dimers of alkyl acrylates using bis-aminophosphines as catalysts.

### Technical Background

The use of specific phosphines as catalysts for the dimerization of alkyl acrylates via the Rauhut-Currier reaction has already been described in the prior art.

US 3074999 A describes an alkyl acrylate dimerization reaction catalyzed by tertiary phosphines having three alkyl groups, three alicyclic groups or three aryl groups such as tributylphosphine or triphenylphosphine. However, these catalysts exhibit a low activity in the dimerization reaction. Regarding the disclosed process, moderate yields are reported which are severe drawbacks for a commercial production.

US 3227745 A describes an alkyl acrylate dimerization reaction catalyzed by a tertiary phosphine in the presence of large amounts of *tert*-butyl alcohol as a solvent. The disclosed tertiary phosphines are trialkylphosphines. However, with the described process only a low conversion of the acrylate of less than 50% is achieved, which is not suitable for industrial production processes.

US 3342853 A describes the acrylate dimerization catalyzed by triaminophosphines that can be generated prior to the dimerization reaction from PCl₃. Yields of 70-80% of the methyleneglutarate ester dimers are reported when the reaction is conducted at 60-65°C, however significant amounts of by-product is also generated. Furthermore, triaminophosphines are generally toxic and CMR reagents (carcinogenic, mutagenic and reprotoxic reagents) and when the catalyst is generated in-situ, PCl₃ is used as the precursor which is a very hazardous chemical. These are serious drawbacks for the commercialization and industrialization of this process.

US 3342854 A describes acrylate dimerization reactions catalyzed either by mono-aminophosphines or bis-aminophosphines. However, the low activity of diphenylaminophosphines for the acrylate dimerization requires the use of high phosphine loadings which is a severe drawback for commercial productions. This is shown by two examples in this patent application using either an in-situ generated dibutylaminodiphenylphosphine catalyst or a diethylaminodiphenylphosphine catalyst which result to dimer yields equal to or less than 10%. Furthermore, using the process according to US 3342854 A a significant amount of by-product is obtained.

Weiping Su et al. describe in "P(RNCH2CH2)3N: Catalysts for the Head-to-Tail Dimerization of Methyl Acrylate" J. Org. Chem. 2003, 68, 9499-9501 methyl acrylate dimerization in THF or dioxane as solvents at room temperature using proazaphosphatranes as phosphine catalysts. At 1 mol% catalyst loading, up to 82% yield is obtained. However, the catalysts described in this paper are quite complex and difficult to synthetize resulting in overall expensive catalysts costs which is a severe drawback for a potential industrialization. Moreover, using low catalyst loadings (1 mol%), the reaction kinetics are slow at room temperature resulting in long reaction times (up to 24h) which is a drawback for industrial production.
Other specific phosphines are described by T. Aran Luiz et al in " Synthesis and X-Ray Structural Characterization of (Diisopropylamino)(morpholino) (phenyl)phosphine and its dimeric copper(I)" Synthesis and Reactivity in Inorganic, Metal-Organic, and Nano-Metal chemistry, 2007, 37:9, 669-675 or by Y. Chevallier et al in "Les aminophosphines dans l'hydrogénation homogene par les catalyseurs au Rhodium" Tetrahedron Lett. 1969, 15, 1197-1200. However none of these 2 documents describe the use of such phosphines as catalysts for dimerization reactions of alkyl acrylates.

### Summary of the invention

It is a problem of the present invention to provide a durable, robust, reusable, inexpensive and readily accessible catalyst for the dimerization reaction of alkyl acrylates. Furthermore, it is a problem of the present invention to provide a catalyst for the dimerization of alkyl acrylates, which is relatively low in toxicity, which can be used with relatively low catalyst loadings and provides excellent selectivity.

Moreover, it is a problem of the present invention to provide an efficient process for preparing the inexpensive catalyst for the dimerization reaction of alkyl acrylates.

In addition, it is a problem of the present invention to provide an efficient process for preparing an alkyl acrylate dimer. Specifically, it is a problem of the present invention to provide a process for preparing an alkyl acrylate dimer, wherein the use of large amounts of tertiary alcohols as solvents and relatively high catalysts loadings can be avoided. More specifically, it is a problem of the present invention to provide an efficient process for preparing a hydrogenated alkyl acrylate dimer and an efficient process for preparing a hydrolyzed alkyl acrylate dimer.

It has now been found that these and other problems can be solved by the catalyst and processes of the present invention. The present invention relates to a compound according to the following formula (I): 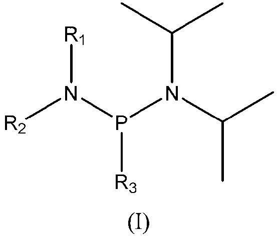 wherein
R₁ and R₂, which are identical or different, are either linear alkyl groups comprising from 1 to 6 carbon atoms or form together with the N atom a heteroaliphatic cycle comprising 3 or 4 carbon atoms; and
R₃ is an aryl or heteroaryl group.

Furthermore, the present invention relates to a process for producing a compound according to formula (I) as defined above, comprising the following steps:
a) reacting a compound according to the following formula (II): 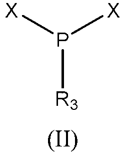
   wherein both X, which are identical or different, are a chloride, a bromide or an iodide and R₃ is as defined as above,
   with diisopropylamine to obtain a haloaminophosphine intermediate according to the following formula (III): 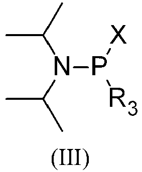
b) reacting the compound of formula (III) as obtained at step a) with an amine of formula (IV): R₁R₂NH (IV), with R₁ and R₂ being as defined above to obtain the compound of formula (I) as defined above.

Additionally, the present invention provides the use of the compound of formula (I) as defined above as catalyst for dimerization reactions of alkyl acrylates.

In addition, the present invention relates to a process comprising a step i) of dimerization of alkyl acrylates according to formula (V) to obtain a dimer according to formula (VI) using the compound according to formula (I) as defined above as catalyst, according to the following reaction scheme: 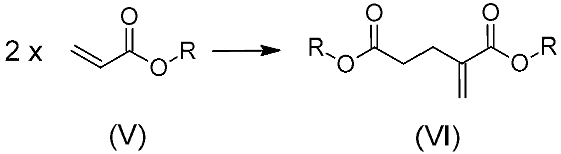 wherein R is an alkyl group.

Additionally, the present invention provides a process comprising step i) as defined above, and further comprising a step ii) of hydrogenation of the dimer according to formula (VI) obtained in the step of dimerization using H₂ and a hydrogenation catalyst, such as Pd based catalysts, Ru based catalysts, Pt based catalysts, Ni based catalysts, Co based catalyst, Rh based catalyst or Ir based catalyst, to obtain a compound according to formula (VII): 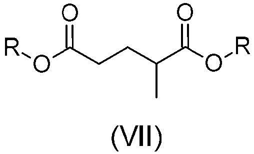 wherein R is as defined above.

Finally, the present invention relates to a process comprising step i) as defined above, further comprising a step ii') of hydrolysis of the dimer according to formula (VI) obtained in the step of dimerization using acid catalysts such as Lewis or Bronsted acids, to obtain a compound according to formula (VIII):

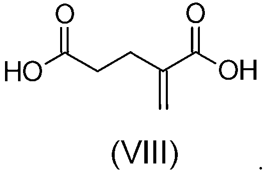

The present invention is based on the recognition that a durable, robust, reusable, inexpensive and readily accessible catalyst for the dimerization reaction of alkyl acrylates is provided in form of the compound according to formula (I). The catalyst for the dimerization of alkyl acrylates according to formula (I) is relatively low in toxicity, reusable, can be used at relatively low catalyst loadings and provides excellent selectivity. Moreover, the present invention provides an efficient and simple process using relatively inexpensive starting materials for preparing the catalyst for the dimerization reaction of alkyl acrylates of the present invention in an inexpensive manner. In addition, the present invention provides an efficient process for preparing an alkyl acrylate dimer using the compound according to formula (I) as a catalyst, wherein the use of large amounts of tertiary alcohols with respect to the alkyl acrylate and the use of relatively high catalyst loadings can be avoided. Specifically, the amount of the tertiary alcohol with respect to the alkyl acrylate can be reduced to a molar ratio of 0.01:1 and the catalyst loading can be reduced to 0.20 mol%. Finally, the present invention provides an efficient process for preparing a hydrogenated alkyl acrylate dimer and an efficient process for preparing a hydrolyzed alkyl acrylate dimer.

### Detailed description of the invention

According to the present invention the term "about" means ±10% of the specified numeric value, preferably ±5% and most preferably ±2%.

The present invention relates to the compound according to the following formula (I): 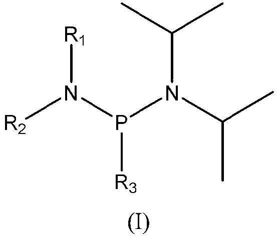 wherein
R₁ and R₂, which are identical or different, are either linear alkyl groups comprising from 1 to 6 carbon atoms or form together with the N atom an heteroaliphatic cycle comprising 3 or 4 carbon atoms; and
R₃ is an aryl or heteroaryl group.

Preferably, the compound according to formula (I) is a compound, wherein R₃ is selected from: phenyl; *ortho-, meta-* or *para-* tolyl; xylyl including all position isomers such as: 2,3-dimethylphenyl, 2,4-dimethylphenyl, 2,5-dimethylphenyl, 2,6-dimethylphenyl, 3,4-dimethylphenyl and 3,5-dimethylphenyl; 3-methyl-4-methoxyphenyl, 2-methyl-4-methoxyphenyl, 2-methyl-3-methoxyphenyl, 4-methyl-3-methoxyphenyl, 5-methyl-3-methoxyphenyl, 6-methyl-3-methoxyphenyl 2-methoxy-3-methylphenyl, 2-methoxy-4-methylphenyl, 2-methoxy-5-methylphenyl and 2-methoxy-6-methylphenyl; mesityl including all position isomers such as: 2,3,4-trimethylphenyl, 2,3,5-trimethylphenyl, 2,3,6-trimethylphenyl, 2,4,5-trimethylphenyl, 2,4,6-trimethylphenyl and 3,4,5-trimethylphenyl; duryl including all position isomers such as: 2,3,4,5-tetramethylphenyl, 2,3,4,6-tetramethylphenyl and 2,3,5,6-tetramethylphenyl; pentamethylphenyl, 2,6-diisopropylphenyl; *ortho-, meta-* or *para- tert*-butylphenyl; 2,3-di-*tert-*butylphenyl, 2,4-di-tert-butylphenyl, 2,5-di-*tert*-butylphenyl, 2,6-di-*tert-*butylphenyl, 3,4-di-*tert*-butylphenyl and 3,5-di-*tert*-butylphenyl; *ortho-, meta-* or *para*-methoxyphenyl; *ortho-, meta-* or *para*-chlorophenyl; 2,3-dimethoxyphenyl, 2,4-dimethoxyphenyl, 2,5-dimethoxyphenyl, 2,6-dimethoxyphenyl, 3,4-dimethoxyphenyl and 3,5-dimethoxyphenyl; 2,3-methylenedioxyphenyl, 3,4-methylenedioxyphenyl; *ortho-, meta-* or *para-* nitrophenyl; *ortho-, meta-* or *para-*biphenyl; *ortho-, meta-* or *para-* trifluoromethylphenyl, *ortho-, meta-* or *para-*fluorophenyl; 1- or 2- naphtyl; 2-pyridyl, 3-pyridyl or 4-pyridyl; 2-furyl, 3-furyl; 1-pyrrolyl, 2-pyrrolyl or 3-pyrrolyl; 2-thiophenyl, 3-thiophenyl; 2-indolyl, 3-indolyl and 2-benzofuryl, 3-benzofuryl, preferably phenyl; *ortho-, meta-* or *para-*tolyl or xylyl and its isomer positions.

More preferably, the compound according formula (I) is a compound, wherein R₃ is selected from: phenyl, tolyl, xylyl, mesityl, duryl, pentamethylphenyl, 2,6-diisopropylphenyl, tert-butylphenyl, ditert-butylphenyl, methoxyphenyl, dimethoxyphenyl, methoxytolyl, methylenedioxyphenyl, biphenyl, nitrophenyl, halogen substituted phenyl, trifluoromethylphenyl, naphtyl, pyridyl, furyl, pyrrolyl, thiophenyl, 2-indolyl, benzofuryl and all their position isomers.

Still more preferably, the compound according to formula (I) is a compound, wherein R₃ is selected from: phenyl, tolyl, xylyl and mesityl. Even more preferably, the compound of formula (I) is a compound, wherein R₃ is selected from: phenyl or tolyl. Most preferably, the compound of formula (I) is a compound, wherein R₃ is phenyl.

Preferably, the compound according to formula (I) is a compound, wherein R₁ and R₂ are identical or different linear alkyl groups comprising from 1 to 6 carbon atoms, preferably from 2 to 6 carbon atoms, more preferably 2 carbon atoms.

Preferably, the compound according to formula (I) is a compound, wherein R₁ and R₂ are identical linear alkyl groups comprising from 1 to 6 carbon atoms, preferably 2 to 6 carbon atoms, still more preferably 2 carbon atoms.

Preferably, the compound according to formula (I) is a compound, wherein R₁ and R₂ are different linear alkyl groups comprising from 1 to 6 carbon atoms, preferably 1 or 2 carbon atoms.

Preferably, the compound according to formula (I) is a compound, wherein R₁ and R₂ form together with the N atom an heteroaliphatic cycle comprising 3 or 4 carbon atoms, preferably 4 carbon atoms. Still more preferably the R₁ and R₂ form together with the N atom an heteroaliphatic cycle comprising 3 or 4 carbon atoms, preferably the heteroaliphatic cycle comprises no other type of heteroatom preferably the heteroaliphatic cycle formed comprises only one heteroatom, which is the N atom.

Preferably, the compound according to formula (I) is a compound, wherein R₃ is a phenyl and R₁ and R₂ form together with the N atom a heteroaliphatic cycle comprising 4 carbon atoms.

Most preferably, the compound according to formula (I) is the following compound (IX):

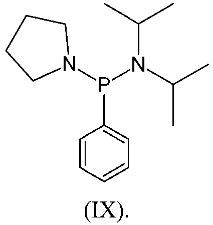

Furthermore, the present invention relates to a process for producing a compound according to formula (I) as defined above, comprising the following steps:
a) reacting a compound according to formula (II): 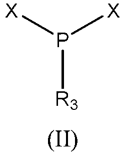
   wherein both X, which are identical or different, are a chloride, a bromide or an iodide and R₃ is as defined above,
   with diisopropylamine to obtain a haloaminophosphine intermediate according to formula (III): 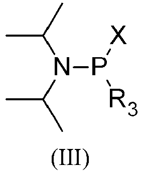
b) reacting the compound according to formula (III) as obtained at step a) with an amine according to formula (IV): R₁R₂NH (IV), with R₁ and R₂ being as defined above to obtain the compound according to formula (I) as defined above.

In a preferred embodiment, in the process for producing a compound according to formula (I) as defined herein, the steps a) and b) are conducted successively in the same reactor.

Preferably, in the process for producing a compound according to formula (I) as defined herein, the steps a) and b) are performed in an organic solvent, which can be the same or different for each step, preferably an aprotic solvent, more preferably selected from tetrahydrofuran (THF), methyl-tetrahydrofuran (MeTHF), toluene, xylene, diethyl ether, *tert*-butyl methyl ether (TBME), dichloromethane (DCM), chloroform, dioxane, hexane, cyclohexane, benzene, anisole and acetonitrile, still more preferably toluene, anisole or MeTHF, most preferably MeTHF and anisole.

Preferably, in the process for producing a compound according to formula (I) as defined herein, the steps a) and b) are performed at a temperature, which can be the same or different for each step, ranging from about 0°C to about 100°C, preferably about 0°C to about 80°C, more preferably about 25 to about 60°C, most preferably about 40°C.

Preferably, in the process for producing a compound according to formula (I) as defined herein, the step a) is carried out by the slow addition of the reactant of formula (II) to a solution of diisopropylamine in the aprotic solvent where diisopropylamine is used in an amount equal to or higher than 2 equivalents with respect to the reactant (II) as it has been surprisingly found that only one halogen X in (II) can be substituted by a diisopropylamino groupment.

Preferably, in the process for producing a compound according to formula (I) as defined herein, the steps a) and b) are both carried out in anhydrous conditions and in the absence of oxygen.

Preferably, in the process for producing a compound according to formula (I) as defined herein, the step b) is followed by a filtration step to remove ammonium chloride salts by-products formed.

Moreover, the present invention relates to the use of the compound according to formula (I) as defined above as catalyst for dimerization reactions of alkyl acrylates.

In addition, the present invention relates to a process comprising a step i) of dimerization of alkyl acrylates according to formula (V) to obtain a dimer according to formula (VI) using the compound according to formula (I) as defined herein as catalyst, according to the following reaction scheme: 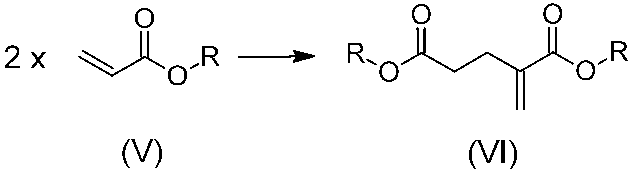 wherein R is an alkyl group.

Preferably, in the process comprising step i) as defined herein, R is a C₁-C₁₈ alkyl such as methyl, ethyl, propyl, isopropyl, n-butyl, *tert*-butyl, *sec*-butyl, isobutyl, pentyl, hexyl, 2-ethylhexyl, octyl, decyl, dodecyl, t-dodecyl, tetradecyl, hexadecyl and octadecyl, preferably a C₁-C₈ alkyl and more preferably a C₁-C₄ alkyl, most preferably a methyl.

Preferably, in the process comprising step i) as defined herein, the step i) of dimerization is performed in an organic solvent, preferably an aprotic solvent, more preferably selected from tetrahydrofuran (THF), methyl-tetrahydrofuran (MeTHF), toluene, xylene, anisole, diethyl ether, tert-butyl methyl ether (TBME), dichloromethane (DCM), chloroform, dioxane, hexane, cyclohexane, benzene and acetonitrile, most preferably MeTHF and anisole.

Preferably, in the process comprising step i) as defined herein, the step i) of dimerization is performed in presence of a co-solvent being a tertiary alcohol or a silanol, preferably a tertiary alcohol, more preferably *tert*-butanol, pinacol or *tert-*amyl alcohol, most preferably *tert*-butanol.

Preferably, in the process comprising step i) as defined herein, the molar ratio co-solvent/alkyl acrylate of formula (V) is from about 4:1 to about 0.01:1, preferably from about 2:1 to about 0.1:1 and more preferably from about 0.5:1 to about 0.1:1.

Preferably, in the process comprising step i) as defined herein, in step i) the compound according to formula (I) as defined herein is used in an amount of from about 0.20 mol% to about 0.90 mol% with respect to the amount of the alkyl acrylate, more preferably of from about 0.25 mol% to 0.80 mol%, still more preferably of from about 0.30 mol% to about 0.70 mol%, even more preferably of from about 0.30 mol% to about 0.50 mol%, even still more preferably of from about 0.30 mol% to about 0.45 mol%, most preferably about 0.33 mol%.

Preferably, in the process comprising step i) as defined herein, the dimerization step i) is performed at a temperature ranging from about 20°C to about 100°C, preferably from about 20°C to about 80°C, more preferably from about 25°C to about 60°C, most preferably about 45°C.

Preferably, in the process comprising step i) as defined herein, the dimerization step i) is carried out in anhydrous conditions and in the absence of oxygen.

Preferably, the process comprising step i) as defined above, further comprises a step ii) of hydrogenation of the dimer according to formula (VI) obtained in the step of dimerization using H₂ and a hydrogenation catalyst, such as Pd based catalysts, for example Pd/C, Pd/Al₂O₃, Pd/SiO₂, Ru based catalysts, for example Ru/C, Pt based catalysts, such as Pt/C, Ni based catalysts, such as supported Nickel or Raney Nickel, Co based catalyst such as supported cobalt or Raney cobalt, Rh based catalyst such as Rh/C, Ir based catalyst such as Ir/C, preferably Pd/C or Raney Nickel, to obtain a compound according to formula (VII): 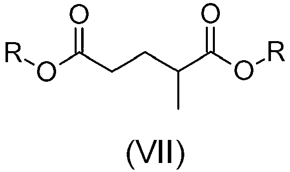 wherein R is as defined above.

In a preferred embodiment, in the process comprising steps i) and ii), the steps i) and ii) are followed by a step ii') of hydrolysis of the hydrogenated dimer according to formula (VII) obtained by the steps of dimerization and hydrogenation using acid catalysts, such as Lewis or Bronsted acids, for example: HCl, H₂SO₄, *para*-toluenesulfonic acid, methanesulfonic acid, triflic acid and solid acidic catalysts, such as Amberlyst resins, zeolites or Nafion, to obtain a compound according to formula (X):

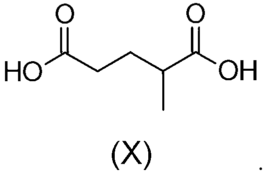

Preferably, the process comprising the step i) as defined above, further comprises a step ii') of hydrolysis of the dimer according to formula (VI) obtained in the step of dimerization using acid catalysts, such as Lewis or Bronsted acids, for example: HCl, H₂SO₄, *para*-toluenesulfonic acid, methanesulfonic acid, triflic acid and solid acidic catalysts, such as Amberlyst resins, zeolites and Nafion, to obtain a compound according to formula (VIII):

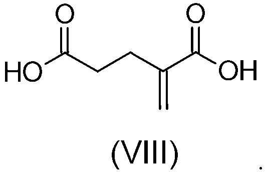

In a preferred embodiment, in the process comprising steps i) and ii'), the steps i) and ii') are followed by a step ii) of hydrogenation of the hydrolyzed dimer according to formula (VIII) obtained by the steps of dimerization and hydrolysis using H₂ and a hydrogenation catalyst, such as Pd based catalysts, for example Pd/C, Pd/Al₂O₃, Pd/SiO₂, Ru based catalysts, for example Ru/C, Pt based catalysts, such as Pt/C, Ni based catalysts, such as supported Nickel or Raney Nickel, Co based catalyst such as supported cobalt or Raney cobalt, Rh based catalyst such as Rh/C, Ir based catalyst such as Ir/C, preferably Pd/C or Raney Nickel, to obtain a compound according to formula (X):

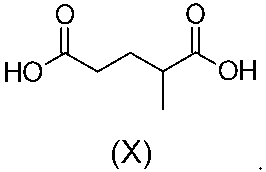

Preferably, the process comprising steps i) and/or ii) and/or ii') further comprises an initial step 0) of preparing the catalyst according to formula (I) as defined above.

In a preferred embodiment, in the process comprising steps i) and/or ii) and/or ii') and further comprising initial step 0), the steps 0) and i) are consecutive steps performed without isolation of the catalyst after step 0).

### Examples

### 1. Aminophosphine catalyzed methyl acrylate dimerization

### Phosphines screening study general protocols:

Different kinds of phosphines were prepared by "in-situ" reaction, meaning that the phosphine catalyst formed was not isolated from the reaction medium before launching the dimerization reaction of methyl acrylate.

### a) Dimerization catalyzed by symmetrical bis-aminophosphines from dichlorophosphines:

All the reactions were conducted in carefully dried vessels under an inert argon atmosphere. Methyl acrylate and *tert*-butanol were dried using 4A molecular sieves and *tert*-butanol was distilled under argon prior to each reaction. Dichlorophosphines and amines were used as such.

In a 25 mL two necked round bottom flask were added:
- 3 mL of 2-methyltetrahydrofuran
- dichlorophosphine precursor (1.8 mmoles, 0.01 eq. with respect to methyl acrylate)

In a 50 mL three necked round bottom flask equipped with a magnetic stirring device were added:
- 1 mL of 2-methyltetrahydrofuran
- 4 equivalents with respect to the dichlorophosphine precursor of the desired amine (7.2 mmoles).

The dichlorophosphine solution was progressively added to the amine solution under stirring (1400 rpm) over 1 hour while keeping the temperature of the reaction medium below 40°C (exothermic reaction). Upon dichlorophosphine addition to the amine solution, there was formation of a white precipitate corresponding to the insoluble ammonium chloride salt by-product. At the end of the addition, the mixture was then allowed to stir at ambient temperature and the reaction progress was monitored thanks to ³¹P NMR (see table 1 below for the results of ³¹P chemical shifts of investigated aminophosphines).

After phosphine formation completion (which usually requires 1h of stirring at room temperature after chlorophosphine addition for the unhindered amines and 2h for the more sterically hindered amines) the mixture was then filtered via cannula into a 100 mL three necked round bottom flask equipped with a magnetic stirrer, a condenser, a heater and a temperature probe and containing 32 mL of melted *tert*-butanol (2:1 v/v with respect to methyl acrylate). The mixture was then allowed to stir at 60°C. Immediately 15.95 mL of methyl acrylate (15.15 g, 0.176 mole, 1eq.) was carefully added over 1 hour (exothermy) into the reactor and the reaction progress was monitored thanks to ¹H NMR. Reactions were pursued until advancement ceased or until 1 day of reaction. The conversion of methyl acrylate was then estimated by ¹H NMR thanks to the integration of the methylene protons of the products and the methylene protons in the starting methyl acrylate.

### NMR spectrum of the product:

**¹H NMR (CDCl₃, 400 MHz) δ (ppm):** 6.03 (s, 1H), 5.46 (s, 1H), 3.60 (s, 3H), 3.51 (s, 3H), 2.48 (t, J=7.6 Hz, 2H), 2.37 (t, J=7.6 Hz, 2H).

### b) Dimerization catalyzed by mono-aminophosphines from mono-chlorophosphines:

All the reactions were conducted in carefully dried vessels under an inert argon atmosphere. Methyl acrylate and *tert*-butanol were dried using 4A molecular sieves and *tert*-butanol was distilled under argon prior to each reaction. Mono-chlorophosphines and amines were used as such.

In a 25 mL two necked round bottom flask are added:
- 3 mL of 2-methyltetrahydrofuran
- Mono-chlorophosphine precursor (1.8 mmoles, 0.01 eq. with respect to methyl acrylate)

In a 50 mL three necked round bottom flask equipped with a magnetic stirring device were added:
- 1 mL of 2-methyltetrahydrofuran
- 2 equivalents with respect to the mono-chlorophosphine precursor of the desired amine (3.6 mmoles).

The mono-chlorophosphine solution was progressively added to the amine solution under stirring (1400 rpm) over 1 hour while keeping the temperature of the reaction medium below 40°C (exothermic reaction). Upon mono-chlorophosphine addition to the amine solution, there was formation of a white precipitate corresponding to the insoluble ammonium chloride salt by-product. At the end of the addition, the mixture was then allowed to stir at ambient temperature and the reaction progress was monitored thanks to ³¹P NMR.

After phosphine formation completion (which usually requires 1h of stirring at room temperature after mono-chlorophosphine addition for the unhindered amines and 2h for the more sterically hindered amines) the mixture was then filtered via cannula into a 100 mL three necked round bottom flask equipped with a magnetic stirrer, a condenser, a heater and a temperature probe and containing 32 mL of melted *tert*-butanol (2:1 v/v with respect to methyl acrylate). The mixture was then allowed to stir at 60°C. Immediately, 15.95 mL of methyl acrylate (15.15 g, 0.176 mole, 1eq.) was carefully added over 1 hour (exothermy) into the reactor and the reaction progress was monitored thanks to ¹H NMR. Reactions were pursued until advancement ceased or until 1 day of reaction. The conversion of methyl acrylate was then estimated by ¹H NMR thanks to the integration of the methylene protons of the products and the methylene proton in the starting methyl acrylate.

### c) Dimerization catalyzed by unsymmetrical bis-aminophosphines from dichlorophosphines, diisopropylamine and an additional amine:

All the reactions were conducted in carefully dried vessels under an inert argon atmosphere. Methyl acrylate and *tert*-butanol were dried using 4A molecular sieves and *tert*-butanol was distilled under argon prior to each reaction. Dichlorophosphines and amines were used as such.

In a 25 mL two necked round bottom flask were added:
- 3 mL of 2-methyltetrahydrofuran
- dichlorophosphine precursor (1.8 mmoles, 0.01 eq. with respect to methyl acrylate)

In a 50mL three necked round bottom flask equipped with a magnetic stirring device were added:
- 1 mL of 2-methyltetrahydrofuran
- 3 equivalents with respect to the dichlorophosphine precursor of diisopropylamine (5.4 mmoles).

The dichlorophosphine solution was progressively added to the amine solution under stirring (1400 rpm) over 1 hour while keeping the temperature of the reaction medium below 40°C (exothermic reaction). Upon dichlorophosphine addition to the amine solution, there was formation of a white precipitate corresponding to the insoluble ammonium chloride salt by-product (in the case of the invention diisopropylammonium chloride). The mixture was then allowed to stir at ambient temperature and the reaction progress was monitored thanks to ³¹P NMR. Formation of the intermediate chloro(diisopropylamino)phosphine was confirmed by ³¹P NMR (for example for the chlorophenyl(diisopropylamino)phosphine a singlet was observed at +132.5 ppm).

After chloroaminophosphine intermediate formation completion (which usually requires 1h of stirring at room temperature after the dichlorophosphine addition), 1 equivalent of a second amine (1.8 mmoles) was added to the mixture at room temperature under stirring and the reaction mass was allowed to stir at room temperature for an additional hour.

After bis-aminophosphine completion, the mixture was then filtered via cannula into a 100 mL three necked round bottom flask equipped with a magnetic stirrer, a condenser, a heater and a temperature probe and containing 32 mL of melted *tert*-butanol (2:1 v/v with respect to methyl acrylate). The mixture was then allowed to stir at 60°C. Immediately, 15.95 mL of methyl acrylate (15.15 g, 0.176 mole, 1eq.) was carefully added over 1 hour (exothermy) into the reactor and the reaction progress was monitored thanks to ¹H NMR. Reactions were pursued until advancement ceased or until 1 day of reaction. The conversion of methyl acrylate was then estimated by ¹H NMR thanks to the integration of the methylene protons of the products and the methylene proton in the starting methyl acrylate.

In order to confirm that the targeted catalysts have been successfully synthesized, the crude reaction medium was analyzed using ³¹P NMR. Indeed, this parameter (³¹P NMR chemical shift) was characteristic for the synthetized aminophosphine and the area under the peak was proportional to the molar concentration of the aminophosphine in solution. The ³¹P NMR spectrum were recorded using a Bruker Avance 400 MHz spectrometer. The NMR yield of the phosphine (%), which corresponds to the molar selectivity of the aminophosphine synthesis reaction deduced from the peak areas in the ³¹P NMR spectra recorded on the Me-THF solution before transfer into the dimerization reactor, was also measured.

All the results in terms of phosphine catalysts and their process of preparation are compiled in table 1 below:

**Table 1. Aminophosphines screening study results - catalyst synthesis**

| **N°** | **Chlorophos phine precursor** | **Amine 1** | **Amine 2** | **³¹p shift (ppm)** | **Chlorophosp hine loading (mol%)** | **NMR yield Phos phine (%)** |
|---|---|---|---|---|---|---|
| Cp 1 | 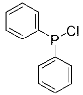 | 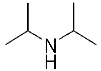 | -- | 37.1 | 1 | 41 |
| Cp 2 | 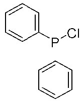 | 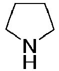 | -- | 46.9 | 1 | **83** |
| Cp 3 | 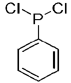 | 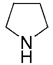 | 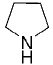 | 72.7 | 1 | **85** |
| Cp 4 | 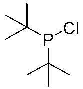 | 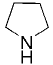 | -- | -- | 1 | 0 |
| Cp 5.1 | 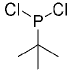 | 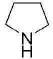 | 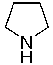 | 97.8 | 1 | 54 |
| Cp 5.2 | 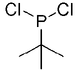 | 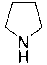 | 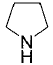 | 97.8 | 0.5 | 46 |
| Inv 1.1 | 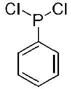 | 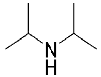 | 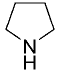 | 63.8 | 1 | **84** |
| Inv 1.2 | 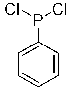 | 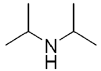 | 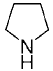 | 63.8 | 0.5 | **96** |
| Inv 1.3 | 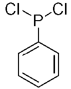 | 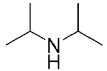 | 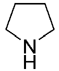 | 63.8 | 0.33 | **98** |
| Inv 1.4 | 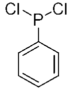 | 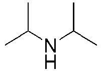 | 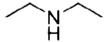 | 77.0 | 0.5 | **98** |
| Inv 2 | 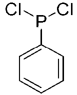 | 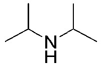 | 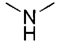 | 78.4 | 0.5 | **95** |

| | | | | | | |
|---|---|---|---|---|---|---|
| (Cp = Comparative Example) | | | | | | |

### Conclusion:

All phosphines were synthesized, except di-*tert*-butylpyrrolidinophosphine (Cp 4); The chlorophosphine precursor was too bulky and cannot accommodate the amine ligand in the substitution reaction under our conditions.

Chlorodiphenylphosphine precursor (Cp 1) gave only moderate yields of the aminophosphine by reaction with the diisopropylamine. Bis-pyrrolidino-*tert-*butylphosphine (Cp 5.1 and 5.2) was quite sensitive toward air and moisture and the phosphine synthesis afforded only moderate yields of the aminophosphine.

The maximum conversion during acrylate dimerization were also measured for some of the trials presented above in table 1, which corresponds to the maximal conversion rate of methyl acrylate measured from ¹H NMR. The ratio of *t*BuOH:Acrylate in v:v (and in mol/mol) is also given.

For Inv 1.3, reaction was started with 0.5 mol% initial dichlorophenylphosphine loading followed by the addition of additional amount of methyl acrylate (0.5 eq. to reach 0.33 mol% initial dichlorophenylphosphine loading) after 20h reaction time.

All the results in terms of dimerization process were compiled in table 2 below:

**Table 2. Aminophosphines screening study results - dimerization process**

| **N°** | **Chlorophos phine precursor** | **Amine 1** | **Amine 2** | **Chlorophos phine loading (mol%)** | **Max. Conversion** | **Ratio tBuOH:Acrylate v:v** | **mol/mol (tBuOH/ Acrylate)** |
|---|---|---|---|---|---|---|---|
| Cp 1 | 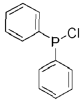 | 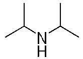 | -- | 1 | 0 | 2:1 | 1.90 |
| Cp 2 | 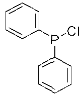 | 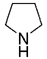 | -- | 1 | 10 | 2:1 | 1.90 |
| Cp 3 | 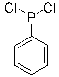 | 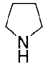 | 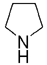 | 1 | 55 | 2:1 | 1.90 |
| Cp 4 | 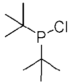 | 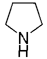 | -- | 1 | 0 | 2:1 | 1.90 |
| Inv 1.1 | 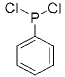 | 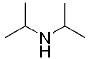 | 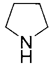 | 1 | 88 | 2:1 | 1.90 |
| Inv 1.2 | 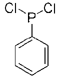 | 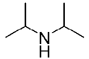 | 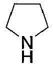 | 0.5 | 76 | 2:1 | 1.90 |
| Inv 1.2bis | 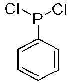 | 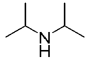 | 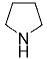 | 0.5 | **84** | 1:1 | 0.95 |
| Inv 1.3 | 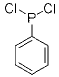 | 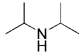 | 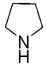 | 0.33 | **91** | 1:4 | 0.24 |
| Inv 1.4 | 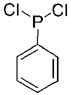 | 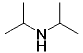 | 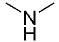 | 0.5 | **43** | 1:4 | 0.24 |

| | | | | | | | |
|---|---|---|---|---|---|---|---|
| (Cp = Comparative Example) | | | | | | | |

### Conclusion:

As it can be seen from table 2, diphenylaminophosphines (Comp 1 and 2) did not afford a good catalytic activity (Max conversion equals zero or 10). Bis-pyrrolidino-phenylphosphine (Comp 3) gave only a moderate conversion of the methyl acrylate (Max conversion of 55). As mentioned previously, Di-*tert-*butylchlorophosphine (Comp 4) was too bulky and cannot accommodate the amine ligand in the substitution reaction thus giving no conversion of the chlorophosphine precursor to the aminophosphine.

On the other hand, the aminophosphines according to the invention (Inv 1.1 to 1.4) provided moderate to good catalytic activities. The best system that displayed the best performance was diisopropylamino-pyrrolidino-phenylphosphine (Inv 1.1 to 1.3).

It was very surprising to observe that at only 0.33 mol% of initial dichlorophosphine loading, an acrylate conversion of 91% was reached with the diisopropylamino-pyrrolidino-phenylphosphine (Inv 1.3). In addition, it was observed that this phosphine was quite robust allowing an easier handling and even allowing its recycling for several batches (see below).

Presence of *tert*-butyl alcohol during the dimerization reaction allowed to improve the selectivity of the reaction toward the expected dimer. Surprisingly it was still possible to find suitable conditions which allowed using *t*-BuOH with very low amounts of basic aminophosphines without compromising the catalytic activity of the phosphine and providing good selectivity.

### d) (diisopropylamino)pyrrolidinophenylphosphine catalyzed (0.4 mol% chlorophosphine precursor with respect to methyl acrylate) methyl acrylate dimerization in tert-butanol (1:8 v/v t-BuOH : methyl acrylate = 0.12 mol/mol), 60 °C with catalyst recycling.

All the reactions were conducted in carefully dried vessels and under an inert argon atmosphere. Methyl acrylate and *tert*-butanol were dried using 4A molecular sieves and *tert*-butanol was distilled under argon prior to each reaction. Dichlorophenylphosphine, diisopropylamine and pyrrolidine were used as such.

In a 50 mL two necked round bottom flask were added:
- 20 mL of 2-methyltetrahydrofuran
- 3.6 mL of dichlorophenylphosphine (4.77 g, 0.027 mole, 0.012 eq.)

In a 100 mL three necked round bottom flask equipped with a magnetic stirring device were added:
- 20 mL of 2-methyltetrahydrofuran
- 11.15 mL of diisopropylamine (8.05 g, 0.08 mole, 0.036 eq.) (3 equivalents with respect to the dichlorophenylphosphine)

The dichlorophenylphosphine in 2-methyltetrahydrofuran solution was progressively added to the amine solution under stirring (1400 rpm) over 1 hour while keeping the temperature of the reaction medium below 40°C (exothermic reaction). The mixture was allowed to stir at room temperature and 1 equivalent (0.027 mole, 1.92 g) of pyrrolidine was added to the reaction mixture which was allowed to stir at room temperature for an additional hour in order to complete bis-(amino)phosphine formation.

The reaction mixture was filtered via cannula into a 500 mL double jacketed reactor equipped with a temperature probe, a condenser and a mechanical stirrer (propeller with four inclined plows) containing:
- 25 mL of distillated *tert*-butanol (1:8 v/v *tert*-butanol : methyl acrylate)
- 200 mL of methyl acrylate (190.1 g, 2.2 moles, 1eq.)

The mixture was then allowed to stir at 60°C during 19 hours. The reaction progress was monitored thanks to ¹H NMR. According to ¹H NMR, the conversion of starting methyl acrylate was ~ 86 mol%. The volatiles (t-BuOH, Me-THF and unconverted methyl acrylate) were distilled off allowing to recover 20 g of methyl acrylate. The desired product (dimethyl 2-methyleneglutarate) was then distilled under vacuum (125°C, 7 mbar) to afford 103 g of analytically pure product.

Then, 190 g of methyl acrylate (2.2 moles, 1 eq.) was added to the residue still containing active phosphine catalyst followed by the addition of 20 g of *tert-*butanol. The mixture was allowed to stir at 60°C for an additional 16 hours in order to convert a second batch of methyl acrylate. Volatiles were distilled off allowing to recover 37 g of methyl acrylate and the product was distilled under vacuum (125°C, 8 mbar) to afford 126 g of analytically pure product.

Finally, an additional 190 g of methyl acrylate (2.2 moles, 1 eq.) was added to the residue which still contained active phosphine and the mixture was stirred again at 70°C during 20 hours. At the end of the reaction the volatiles were removed under vacuum to recover 44 g of methyl acrylate, and the product was distilled under vacuum to afford 91 g of pure product.

In total, 320 g of dimethyl 2-methyleneglutarate product was recovered corresponding to a global isolated purified yield of 56 %.

This is the first example of an aminophosphine catalyst that can be recycled after acrylate dimerization reaction.

### e) Influence of the presence/absence of t-BuOH

All the reactions were conducted in carefully dried vessels under an inert argon atmosphere. Methyl acrylate were dried using 4A molecular sieves. Dichlorophosphines and amines were used as such.

In a 25 mL two necked round bottom flask were added:
- 18 mL of 2-methyltetrahydrofuran
- dichlorophosphine precursor (10.5 mmoles, 0.005 eq. with respect to methyl acrylate)

In a 50mL three necked round bottom flask equipped with a magnetic stirring device were added:
- 6 mL of 2-methyltetrahydrofuran
- 3 equivalents with respect to the dichlorophosphine precursor of diisopropylamine (31.4 mmoles).

The dichlorophosphine solution was progressively added to the amine solution under stirring (1400 rpm) over 1 hour while keeping the temperature of the reaction medium below 40°C (exothermic reaction). Upon dichlorophosphine addition to the amine solution, there was formation of a white precipitate corresponding to the insoluble ammonium chloride salt by-product. The mixture was then allowed to stir at ambient temperature and the reaction progress was monitored thanks to ³¹P NMR. Formation of the intermediate chloro(diisopropylamino)phosphine was confirmed by ³¹P NMR (for example for the chlorophenyl(diisopropylamino)phosphine a singlet was observed at +132.5 ppm).

After chloroaminophosphine intermediate formation completion (which usually requires 1h of stirring at room temperature after the dichlorophosphine addition), 1 equivalent of pyrrolidine (10.5 mmoles) was added to the mixture at room temperature under stirring and the reaction mass was allowed to stir at room temperature for an additional hour.

After bis-aminophosphine completion, the mixture was then filtered via cannula into a 500 mL double-jacketed reactor equipped with a mechanical stirrer (propeller with four inclined plows), a condenser, a heater and a temperature probe and containing 190 mL methyl acrylate (180 g, 2.1 moles). The mixture was then allowed to stir at 60°C during 20 hours. The conversion of methyl acrylate was then estimated by ¹H NMR thanks to the integration of the methylene protons of the products and the methylene proton in the starting methyl acrylate.

At 66 % methyl acrylate conversion, the selectivity towards dimer as determined by ¹H NMR was estimated to be 78 mol%.

In comparison when *t*-BuOH was present during the reaction (1: 1 v/v tert-BuOH : methyl acrylate), 0.5 mol% dichlorophenylphosphine initial loading, 60°C (corresponding to Inv 1.2bis), at similar conversion level (66 %), the selectivity toward dimer was 87% demonstrating clearly the positive impact of *t-*BuOH on the reaction selectivity.

### 2. Dimethyl 2-methyleneglutarate catalytic hydrogenation to dimethyl 2-methylglutarate

The substrate dimethyl 2-methyleneglutarate (50 g, 0.29 mole) obtained according to the dimerization reaction described above (Inv 1.3) was first added into a 100 mL autoclave reactor equipped with a mechanical stirrer (Rushton turbine) followed by the addition of the Pd/C (3%) catalyst (powder, 51% moisture content, 1g wet corresponding to 0.49 g dry, 1 wt% with respect to the substrate). The reactor was then tightly sealed and was purged 3 times with 20 bar of nitrogen followed by 3 times with 5 bar of hydrogen. The reaction mixture was allowed to stir at 1400 rpm and the temperature of the reaction mixture was then set at 40°C. The reaction medium was then allowed to stir at 40°C, 5 bar hydrogen pressure (1400 rpm) during 6 hours and hydrogen consumption was followed over time.

At the end of the reaction which was confirmed by the absence of hydrogen consumption, the reaction mixture was allowed to cool down at room temperature, stirring was stopped and the autoclave was depressurized. The reactor was purged with nitrogen, the crude was removed from the reactor and the catalyst was removed through filtration. The product dimethyl 2-methylglutarate was obtained after catalyst filtration as a clear liquid (50 g corresponding to a yield of 99%) and was used as such.

### 3. Synthesis of 2-methylenepentanedioic acid from Dimethyl 2-methylenepentanedioate

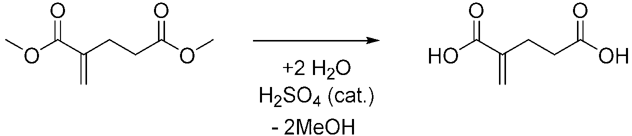

In a 2 L double-jacketed reactor equipped with a mechanical stirrer (propeller with four inclined plows), baffles, a temperature probe and a distillation column connected to a receiver are added:
- 700 g (4.07 mol, 1 eq.) of dimethyl 2-methylenepentanedioate.
- 879 mL of water (48.8 mol, 12 eq).
- Sulfuric acid 95% (9 mL, 16.6 g, 0.163 mole, 4 mol% with respect to dimethyl 2-methylenepentanedioate) which is added drop-wise into the reaction mixture at room temperature thanks to an addition funnel.

The mixture is then stirred at 120°C and the reaction progress is followed by ¹H NMR analysis. Over the course of the reaction, generated methanol is distilled out from the reaction media in order to displace reaction equilibrium toward the desired methyleneglutaric acid.

After 2h30 stirring at 120°C, ¹H NMR analysis indicates a slow conversion of the diester to the diacid, therefor additional amount of sulfuric acid is added into the reaction mixture to speed up the kinetics: 2.26 mL (0.04 mol, 1 mol%) and the temperature of the mixture is increased to 130°C.

However, after 2h additional stirring at 130°C the conversion of the diester is still too slow, therefor 2.26 mL (0.04 mol, 1 mol%) of H₂SO₄ is again added into the reaction mass.

After 11h30 stirring at 130°C, 1060 mL of a water/MeOH mixture were distilled off and 50 mL of fresh water is added into the reaction vessel.

After 16h30 stirring at 130°C, ¹H NMR analysis indicates around 10 mol% of remaining unhydrolyzed ester functions and significant formation of polymer by-products.

At this stage the recovered distillate mass is 1195 g containing 6 g of insoluble starting diester.

The temperature of the reaction medium is lowered to 80°C and 36 mL of a 35 wt% NaOH aqueous solution (2 eq. with respect to H₂SO₄) is slowly added into the vessel to neutralize the catalyst (exothermy).

The reactor content maintained at 80°C is drained into a beaker while constantly stirring and the mixture solidifies into an increasingly firm white paste on cooling. 147 mL of water are added to the paste in order to have a filterable liquid paste and the mixture is allowed to cool down at room temperature to complete precipitation of the diacid product.

The product is then filtered through a sintered filter and a very viscous filtrate is obtained.

The cake is washed 4 times with 80 mL of water then 6 times with 70 mL of water, shaking the mixture well before each filtration.

The resulting aqueous filtrate which has precipitated at room temperature overnight is filtered and rewashed 10 times with 20 mL of water to collect additional product.

The solid fractions are gathered and the product is dried under vacuum at 50°C (10 mbar) for 2 hours to afford 286 g of a white powder with over 98 wt% organic purity and containing 3 wt% water corresponding to 48% isolated yield.

### NMR spectra:

¹H NMR (MeOD-d₄, 400 MHz) δ (ppm): 6.16 (s, 1H), 5.63 (s, 1H), 2.6-2.56 (t, J=7.6 Hz, 2H), 2.51-2.47 (t, J=7.6 Hz, 2H).

¹³C NMR (MeOD-d₄, 101 MHz) δ (ppm): 176.7, 170.12, 141.16, 126.38, 34.07, 28.52.

## Claims

1. Compound according to the following formula (I): 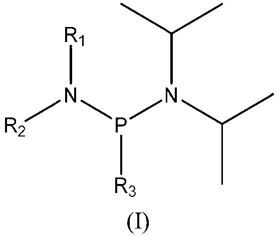 wherein
R₁ and R₂, which are identical or different, are either linear alkyl groups comprising from 1 to 6 carbon atoms or form together with the N atom an heteroaliphatic cycle comprising 3 or 4 carbon atoms; and
R₃ is an aryl or heteroaryl group.

2. Compound according to formula (I) according to claim 1, wherein R₃ is selected from: phenyl, tolyl, xylyl, mesityl, duryl, pentamethylphenyl, 2,6-diisopropylphenyl, tert-butylphenyl, ditert-butylphenyl, methoxyphenyl, dimethoxyphenyl, methoxytolyl, methylenedioxyphenyl, biphenyl, nitrophenyl, halogen substituted phenyl, trifluoromethylphenyl, naphtyl, pyridyl, furyl, pyrrolyl, thiophenyl, 2-indolyl, benzofuryl and all their position isomers.

3. Compound according to formula (I) according to claim 1 or 2, wherein R₁ and R₂ are identical linear alkyl groups comprising from 1 to 6 carbon atoms, preferably 2 to 6 carbon atoms.

4. Compound according to formula (I) according to claim 1 or 2, wherein R₁ and R₂ form together with the N atom an heteroaliphatic cycle comprising 3 or 4 carbon atoms, preferably 4 carbon atoms, and wherein the heteroaliphatic cycle comprises no other type of heteroatom preferably the heteroaliphatic cycle formed comprises only one heteroatom, which is the N atom.

5. Process for producing a compound according to formula (I) as defined in any one of claims 1 to 4, comprising the following steps:
a) reacting a compound according to formula (II): 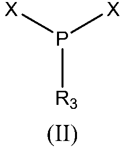
wherein both X, which are identical or different, are a chloride, a bromide or an iodide and R₃ is as defined in claim 1 or 2,
with diisopropylamine to obtain a haloaminophosphine intermediate according to formula (III): 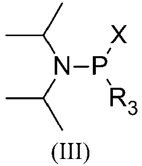
b) reacting the compound according to formula (III) as obtained at step a) with an amine according to formula (IV): R₁R₂NH (IV), with R₁ and R₂ being as defined in any one of claims 1, 3 or 4 to obtain the compound according to formula (I) as defined in any one of claims 1 to 4.

6. Process according to claim 5, wherein the steps a) and b) are performed in an organic solvent, which can be the same or different for each step, preferably an aprotic solvent, more preferably selected from tetrahydrofuran (THF), methyl-tetrahydrofuran (MeTHF), toluene, xylene, anisole, diethyl ether, tert-butyl methyl ether (TBME), dichloromethane (DCM), chloroform, dioxane, hexane, cyclohexane, benzene and acetonitrile, more preferably toluene or MeTHF.

7. Process according to claim 5 or 6, wherein the step b) is followed by a filtration step to remove ammonium chloride salt by-products formed.
